# EUROPEAN PATENT SPECIFICATION

(11) **EP 0 430 511 B1**
(45) Date of publication and mention of the grant of the patent: **07.02.2001**
(21) Application number: 90312498.0
(22) Date of filing: 16.11.1990
(51) Int. Cl.: C12N 15/82, C12N 15/87, C12N 15/54, A01H 5/00

(54) **Soybean plants resistant to glutamine synthetase inhibitors**
Gegen Glutaminsynthetaseinhibitoren resistente Sojabohnenpflanzen
Plantes de soja résistantes aux inhibiteurs de la synthétase de glutamine

(30) Priority: 17.11.1989 US 438650
(43) Date of publication of application: 05.06.1991
(73) Proprietor: MONSANTO COMPANY, St. Louis, Missouri 63167 (US)
(72) Inventor: Russell, David R., Madison, Wisconsin 53705 (US); Swain, William F., Madison, Wisconsin 53705 (US); Fuller, James T., Madison, Wisconsin 53703 (US)
(74) Representative: Bentham, Stephen

(56) References cited:
- EP-A- 0 239 801
- EP-A- 0 242 246
- EP-A- 0 301 749
- TIBTECH, vol. 8, no. 6, June 1990, pages 145-151, Elsevier Science Publishers Ltd; P. CHRISTOU et al.: "Soybean genetic engineering - commercial production of transgenic plants"
- CHEMICAL ABSTRACTS, vol. 112, 1990, page 116, abstract no. 16874e, Columbus, Ohio, US; J. BOTTERMAN et al.: "Discovery, transfer to crops, expression and biological significance of a bialaphos resistance gene", & BCPC MONOGR. 1989, 42(PROSPECTS AMINO ACID BIOSYNTH. INHIB. CROP PROT. PHARM. CHEM.), 63-8
- MOL. GEN. GENET., vol. 217, June 1989, pages 263-268, Springer-Verlag; P. ECKES et al.: "Overproduction of alfalfa glutamine synthetase in transgenic tobacco plants"

## Description

The present invention relates to the creation of genetically engineered plants and, in particular, to the creation of genetically engineered soybean plants and lines conferred with an inheritable trait of resistance to glutamine synthetase inhibitors.

The technology of altering the genetic character of organisms through the application of recombinant DNA technology has been extended in recent years to plants. The technology of creating transgenic plants and lines offers the potential to create novel useful plants in a much more controlled, and at the same time much more rapid, manner than the previously used techniques of more conventional plant breeding. Perhaps even more importantly, the technology of recombinant DNA opens the door to the genetic pantry, in effect, since it makes available for introduction into useful plant crops not just genetic traits native to that species, but also genetic traits found anywhere else in the enormously diverse genetic pool of all the life of the planet. In a noteworthy example, the gene coding for the Lepidopteran-specific protein toxin produced by the common soil bacterium Bacillus thuringiensis has been transferred into the germ line of tobacco and of cotton to yield plants which are thereby rendered toxic to feeding caterpillars.

The advent of the first genetically engineered plants has not led immediately to the creation of transgenic plants of all crop species, however. The first genetically engineered plants, principally tobacco, were produced by taking advantage of the unique method of pathogenicity of another soil dwelling bacterium, Agrobacterium tumefaciens. A. tumefaciens acts on plant hosts by transferring a portion of its DNA, referred to as the T-DNA, into the genome of cells of the host plant to induce those cells to produce a unique class of substances, opines, on which the bacterium feeds. By altering the T-DNA of an Agrobacterium, it is possible to use the bacterium as a vector to transfer foreign DNA into a plant's cells. This method is effective for plants which are suitable hosts for Agrobacterium, as all dicot plants appear to be, and which can be regenerated from a single transformed cell into a whole sexually mature plant. The application of this technique to other plant species has proven difficult at times, however.

One problem in the use of Agrobacterium transformation method or is the need for a selectable marker. In any transformation process in which less than all of the treated cells are transformed, it is necessary to selected the transformant cells or tissues from the non-transformant. While selectable markers from bacteria, such as the aminoglycoside-3-phosphotransferase II (APH II) (also known as neomycin phosphotransferase) gene, have proven effective as selectable markers in tobacco and other model species, it does not provide convenient selection in other important crops. In particular, it is difficult to use effectively as a selectable marker in soybean. Only recently have the first, very few, transgenic soybean plants transformed by Agrobacterium-mediated transformation been reported. Hinchee et al., BioTechnology, 6:915-922 (1988). The use of this method would be thus facilitated by a more efficient selectable marker.

Recently another technology has emerged with great promise for the genetic engineering of plants. This technology involves the physical delivery of DNA into plant cells. The DNA is prepared to properly express in plant cells, and is then coated onto small gold particles which are themselves layered onto a carrier sheet. The carrier sheet is then accelerated by an electric discharge apparatus, the carrier sheet is stopped, and the particles hurl themselves at a suitably placed plant tissue target. The particles are sufficiently small to penetrate the plant's cells without killing them. An apparatus for use in this procedure is described in McCabe et al., published European Patent Applications No. 0270356 and 301 749. The use of this method and apparatus offers the possibility of genetically engineering differentiated plant tissue, such as meristematic tissue, thus avoiding the difficulties and delays of plant regeneration in tissue culture which is inherent in Agrobacterium-mediated transformation.

Once it becomes feasible to genetically engineer crop plants, a logical area of inquiry is directed toward the identification of genes which may be usefully transformed into important crop plants. While yield enhancement is an obvious objective, the genes which condition plant vigor and growth are poorly understood and characterized. Therefore another possible first target are genes which imbue transgenic plants with resistances to factors which would detract from crop yield, such as insects or disease. A related objective is to confer resistance to selective broad-spectrum herbicides, which can then be used to rid farm fields of competing plants, leaving only the transgenic crop plants.

One type of such broad spectrum herbicide is the group of glutamine synthetase inhibitors. These herbicides exhibit toxicity to plants by inactivating the enzyme glutamine synthetase in plant cells, which leads to a build up in these cells of toxic levels of ammonia. Two such herbicides are produced commercially, the herbicide bialaphos, a tripeptide produced by the fungus Streptomyces hygroscopicus and sold under the trademark "Herbiace" by Meija Sieka Ltd., and the herbicide phosphinothricin or PPT, a synthesized organic sold under the trademark "Basta" by Hoechst AG.

The transformation of tobacco, tomato, potato, alfalfa, oil seed rape and sugar beet plants with the Bar gene to confer resistance to enzymes which inhibit glutamine synthetase has been disclosed (Botterman and Leemans, Chemical Abstract, Vol. 112, 1990, abstract 16874). The use of genes coding for resistance to glutamine synthetase inhibitors to confer resistance to herbicides which act by inhibiting glutamine synthetase in a plant cell which is otherwise sensitive to glutamine synthetase has also been disclosed in EP-A-239,801.

The present invention provides a method of genetically engineering a soybean plant for resistance to a glutamine synthetase inhibitor comprising the steps of
(a) isolating a protein coding sequence from a gene for resistance to a glutamine synthetase inhibitor from a microorganism;
(b) joining the protein coding sequence to a flanking regulatory sequence to make a chimeric gene construction effective to express the coding sequence in soybean cells;
(c) coating copies of the chimeric gene construction onto small carrier particles;
(d) physically accelerating the small carrier particles with the chimeric gene construction thereon into soybean tissue which can be cultivated into whole soybean plants;
(e) cultivating the soybean tissue on a medium containing an effective quantity of a glutamine synthetase inhibitor sufficient to be toxic to soybean tissue which is not transformed;
(f) cultivating the surviving tissue into whole soybean plants; and
(g) verifying the presence of the resistance trait in the soybean plants.

In a particular embodiment, the flanking regulatory sequence joined to the coding sequence includes a promoter, a non-translated expression enhancer located 5' to the coding sequence and a polyadenylation sequence located 3' to the coding sequence.

In a further particular embodiment, the step of physically accelerating the particles is performed on an apparatus in which the propulsive force is provided by a spark discharge of electrical energy, with the voltage of the discharge being adjustable to adjust the force by which the particles are accelerated.

The invention further provides a soybean plant comprising in its genome an inheritable trait for resistance to a glutamine synthetase inhibitor and a soybean plant comprising in its genome the Bar gene from Streptomyces hygroscopicus. Seeds of such soybean plants are a further aspect of the invention.

Fig. 1 is a schematic drawing of an apparatus useful for performing the transformation process to produce the plants of the present invention.

Fig. 2 is a top plan view of the spark discharge chamber of the apparatus of Fig. 1.

Fig. 3 illustrates schematically the plasmid manipulations performed to create the plasmid pBar 1.

Fig. 4 illustrates schematically the plasmid manipulations performed to create the plasmid pCMC2060.

Fig. 5 illustrates schematically the plasmid manipulations performed to create the plasmid pCMC2058.

Fig. 6 illustrates schematically the plasmid manipulations performed to create the plasmid pCMC2114.

The practice of the present invention requires both a system for the genetic transformation of soybean and a gene conferring a trait of resistance to glutamine synthetase inhibitors. The method of genetically transforming soybean described below is based on the method disclosed in McCabe et al., Bio/Technology 6:923-926 (1988), the disclosure of which is hereby incorporated by reference. The gene for resistance to glutamine synthetase inhibitors exemplified here is the "Bar" gene which encodes an enzyme referred to as phosphinothricin acetyltransferase or PAT. The enzyme PAT inactivates phosphinothricin, or PPT, by acetylating its free amino group, and the acetylated PPT is unable to inactivate the glutamine synthetase. The Bar gene, found in Streptomyces hygroscopicus has been previously mapped by DeBlock et al., EMBO, 6:2513-2518 (1987) and EP-A-242 246.

The present invention is thus directed toward inserting this fungal gene coding region into a chimeric gene construction effective for expressing the coding region in soybean, and to transforming that chimeric gene construction into the germ line of soybean plants. This process involved significant uncertainty. The effectiveness of the PAT gene to confer resistance in vivo in soybean was uncertain. Both the expression of a foreign gene in soybean and the effectiveness of the enzyme in an in vivo soybean system have not been demonstrated. However, it has been discovered here that the Bar gene can be expressed effectively in soybean cells, that the PAT enzyme does confer resistance to PPT in vivo in soybean, and that PPT can be used as an effective selection agent for transformed cells expressing the Bar gene, even when those cells are a part of an organized plant tissue which is highly chimeric.

To begin the process of the present invention, the gene for resistance to glutamine synthetase inhibition must be isolated and its protein coding sequence removed. This may be most conveniently done by recovering the Bar gene from Streptomyces hygroscopicus. A host strain carrying this trait is available from the American Type Culture Collection (ATCC), Accession No. 21705. The mapping information for the gene has been published. DeBlock et al., EMBO, 6:2513-2518 (1987) and Thompson et al., EMBO, 6:2519-2523 (1987).

The coding sequence obtained from that organism, or from any other source, must then be placed in a chimeric plant expression vector capable of expressing the coding sequence in plant cells. This may be accomplished either by constructing appropriate promoter and terminator sequences and annealing them to opposite ends of the coding sequence, or by inserting the coding sequence in a previously constructed plant expression vector, in a manner well known to the art. Suitable expression promoters, also well known to the art, including the nopaline synthase promoter from Agrobacterium tumefaciens and the CaMV 35S promoter from the 35S sub-unit gene of the cauliflower mosaic virus. One commonly used terminator is the polyadenylation sequence from the nopaline synthase gene from A. tumefaciens. Another useful termination sequence is derived from the soybean small subunit of the carboxylate gene (soybean SSU 3'). Other effective termination regions are known to those of ordinary skill in the art. It has also been discovered that certain non-coding sequences located 5' of the protein coding region enhance expression of the downstream coding region in plants. One such 5' non-coding region is the 5' non-coding leader derived from the alfalfa mosaic virus. These sequences have all been found to function in soybean. In any event, whether these or other equivalent sequences known to those of ordinary skill in this art are used, the coding sequence is joined to appropriate flanking regulatory sequences effective to express the coding sequence in soybean cells.

In transformation experiments, as discussed above, identifying transformant cells or tissues can be an important part of the process. While a selectable marker is preferred, in the absence of a marker which can chemically or biologically select for transformed tissue, a detectable marker which can be discovered by a relatively simple assay is next in preference. Using such a detectable marker, the plant genetic engineer can examine the fate of genes introduced into plants and trace their passage through plant tissues and into progeny. One convenient such detectable marker is the gene for beta-glucuronidase or gus. If expressed in plant tissues, the gus gene will turn X-gluc (5-bromo-4-chloro-3-indolyl glucuronide) blue in a destructive in situ assay. The gus gene has been previously demonstrated to be effectively expressed and detectable in soybean.

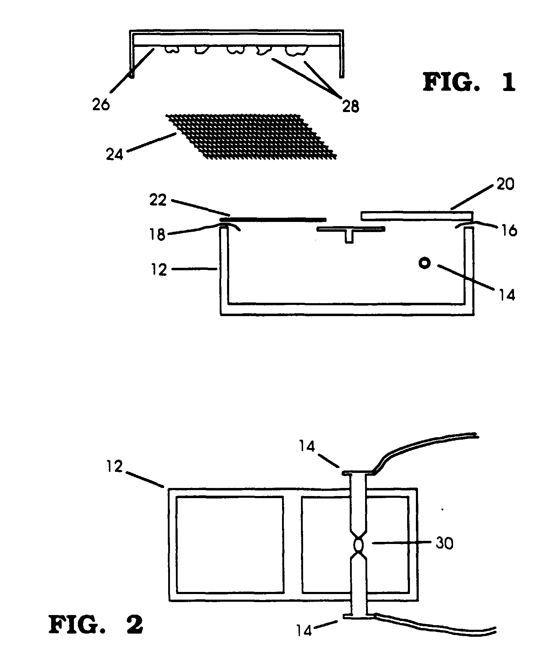

A chimeric gene can be most efficiently transformed into soybean by procedures based on the method disclosed in McCabe, et al., Bio/Technology, 6:923-926 (1988) incorporated by reference above. Shown in Figs. 1 and 2 is the apparatus for use in such a transformation. The transformation apparatus, generally indicated at 10, also referred to as a spark discharge particle accelerator, includes a spark chamber 12 into which are positioned a pair of electrodes 14 the ends of which are spaced closely apart. The electrodes 14 are connected to a source of electric energy (not shown) which is capable of delivering very short discharges of high voltage electrical energy, from the discharge of a capacitor in the single farad range, with the voltage of the discharge being adjustable (by autotransformer) through the range of 1-50 kilovolts. The spark chamber 12 has a first opening 16 over which is positioned an access cover 20. The spark chamber 12 has a second opening 18 over which a carrier sheet 22 of aluminized mylar or similar planar sheet material is placed. The carrier beads, of micron size, and preferably 1 to 3 micron size gold beads, are coated with DNA of the chimeric expression gene, air-dried, and then coated onto the upper face of the carrier sheet 22. Located above the carrier sheet 22, approximately 20 mm away, is a retaining screen 24. Located further above the retaining screen 24, in the range of 20 to 30 mm above the top of the spark chamber 12, is the target 26 in the form of a petrie dish or other container in which the tissue 28 to be transformed has been placed. The entire apparatus is placed in a vacuum chamber (not shown) and evacuated to 500 mm of mercury. Helium gas is bled into the vacuum chamber to replace air inside the vacuum chamber.

In the operation of the apparatus of Fig. 1, a droplet of about 6 microliters of pure distilled water is placed between the ends of the electrodes 14. The access cover 20 can be removed to place the water droplet and then replaced. The carrier sheet 22 carrying gold beads with DNA is then placed over the opening 18 in the top of the spark chamber. The vacuum chamber is then evacuated and the helium introduced. The source of electric energy is then switched resulting in a high voltage discharge between the electrodes 14, vaporizing the water droplet 30, and creating a shock wave inside the spark chamber 12. The shock wave propels the carrier sheet 22 upward until it contacts the retaining screen 24 where it stops. The gold particles are carried by their momentum off the carrier sheet 22 and into the target tissues 28. The transformed tissues may then be removed from the apparatus.

A variety of plant tissue types may be transformed by the apparatus of Figs. 1 and 2. Because the force of the discharge may be adjusted, by adjusting the voltage of the spark discharge, the force with which the particles are driven into the target tissues is adjustable over a significant range. Thus the depth and amount of penetration can be adjusted depending on the hardness and sensitivity of the tissues being transformed. This apparatus may thus be used to transform a very wide variety of tissue types, including seeds, pollen, embryos, callus culture, protoplasts or any differentiated plant tissue. Since the efficient creation of a whole mature plant is usually what is desired, a useful target tissue is the growing meristematic tissue of a seedling or embryo. Such a target, once transformed, can readily be cultivated into a mature soybean plant.

One aspect of the accelerated particle transformation process is that the target tissue, when transformed, is not uniformly transformed. The result, more often than not, is a chimeric plant expressing the introduced gene in sectors or sections of the plant, and not in others. This result is logical given the fact that a region of cells, i.e. a meristem, is transformed, rather than a single cell, and only a statistical fraction of the meristematic cells are transformed. The chimerism of the the plant regenerated from the target tissue, referred to as an RO plant, does not mean completely transformed plants will not result. By allowing the chimeric RO plant to grow to maturity and self-pollinate, R1 offspring will be created, all of which will be clonal or non-chimeric, and some proportion of which may carry the introduced gene. Suitable screening assays must therefore be conducted to identify those R1 plants. An assay useful for the assay of small tissue segments for the presence of a foreign gene is the polymerase chain reaction (PCR) assay as described by Mullis et al. in U.S. Patents No. 4,683,195 and No. 4,683,202.

### Example

### A. Construction of pCMC2114.

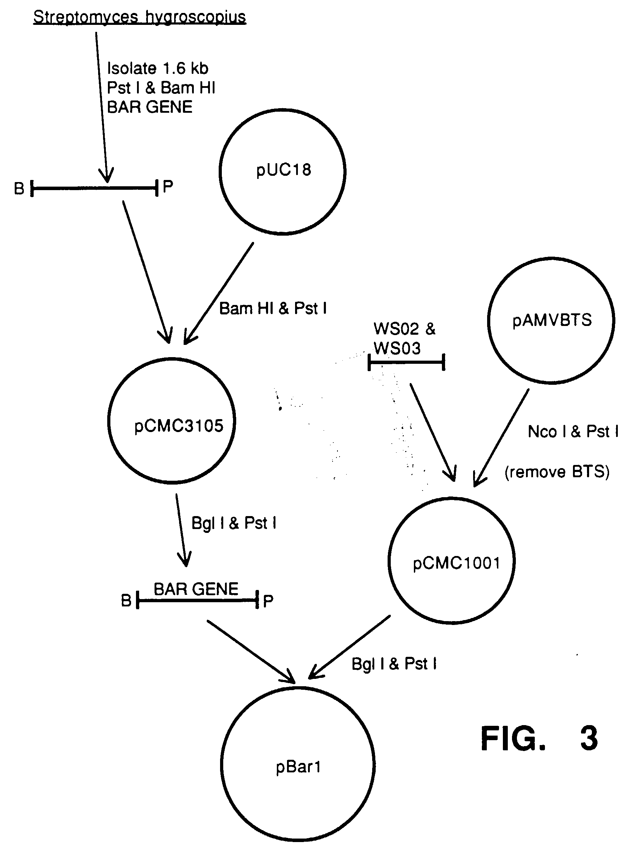

The Bar gene was isolated from Streptomyces hygroscopicus (ATCC 21705) based on the mapping information from DeBlock et al., supra and Thompson et al., supra. The gene was isolated from the fungal DNA as a 1.6 Kb fragment resulting from digestion with Bam HI and Pst I. This fragment was then ligated into a pUC18 vector which had also been digested with Bam HI and Pst I. The presence of the correct fragment was verified by transforming the ligated DNA into E. coli and selection for Bar resistance on a selective media. The plasmid, the derivation of which is schematically illustrated in Fig. 3, was designated pCMC3105.

In order to join the excised Bar gene to a convenient plant expression cassette vector, it was desired to place an Nco I site at the start of the protein coding sequence. This was done by synthesizing a pair of overlapping partially complementary oligonucleotides which were formed into a duplex containing: an Nco I site at the 5' end, a portion of the Bar coding sequence to the Bgl I site naturally occurring in the native sequence, and a Pst I site at the 3' end of the synthetic duplex. the duplex has the following sequence.

5'-CATGGTGAGCCCAGAACGACGCCCGGCCGACATC-
3'-    CACTCGGGTCTTGCTGCGGGCCGGCTGTAG-
-CGCCGTGCCACCGAGGCGAGATCTGATAGTAACTGCA-3'
-GCGGCACGGTGGCTCCGCTCTAGACTATCATTG-5'

This duplex DNA was then inserted into a portion of a plant expression cassette plasmid pAMVBTS, which contains a chimeric plant expression gene including the cauliflower mosaic virus 35S promoter (CaMV35S), the 5' non-coding leader sequence from alfalfa mosaic virus, the coding region for the B.t. (Bacillus thuringiensis) toxin protein, and the polyadenylation region from A. tumefaciens. By digestion with Nco I and Pst I, the B.t. coding sequence was excised from the plasmid. The purified remainder of the plasmid was then joined to the synthetic duplex to form a plasmid, designated pCMC1001, which is similar to pAMVBTS except for the substitution of the 5' segment of the Bar coding region for the B.t. sequence. At the 3' end of the partial Bar coding region of this plasmid are Bgl I, Bgl II and Pst I sites, prior to the termination sequence. The plasmid was then cleaved with both Bgl I and Pst I, and the Bgl I and Pst I fragment from the Bar gene carrier pCMC3105 was inserted into it. This plasmid was designated pBarl. Transient assays in plant cells demonstrated the functionality of the gene to express this coding sequence.

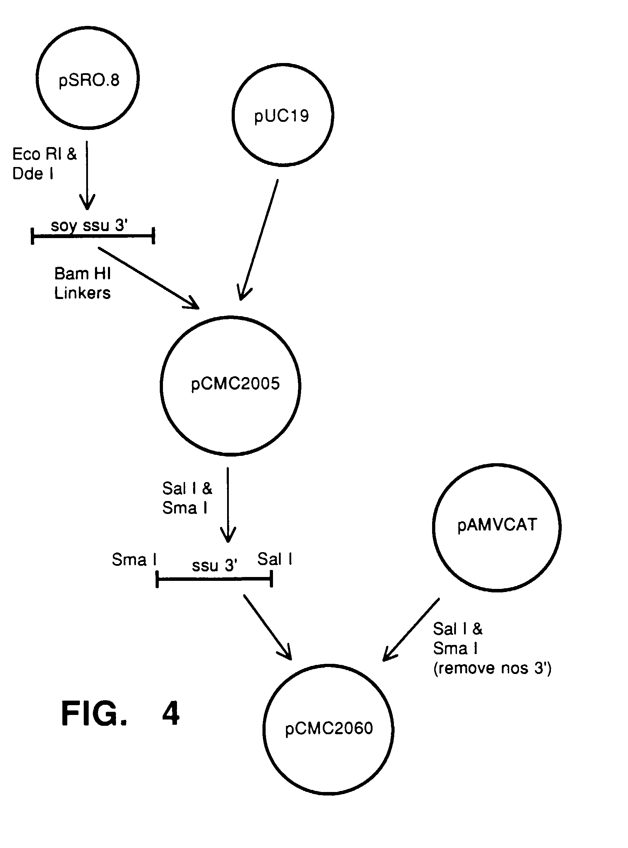

This gene construction was then altered to replace the polyadenylation sequence from nopaline synthase with the termination sequence from a soybean ssu gene. A plasmid pSRO.8 as described in Berry-Lowe et al., J. Mol. and Appl. Genet., 1:483-498 (1982) was obtained. The termination fragment was first isolated as a 300 base pair fragment resulting from Eco RI and Dde I digestion of pSRO.8. This process is illustrated in Fig. 4. This fragment was then made blunt-ended by Klenow polymerase and Bam HI linkers were attached. The fragment was then cut with Bam HI and inserted into the Bam HI site of pUC19 to produce a plasmid designated pCMC2005. This manipulation placed the soybean ssu termination sequence in the same orientation as the nopaline synthase terminator in pBar1 relative to Sma I and Sal I sites which bracket it. The Sma I and Sal I fragment was then isolated from pCMC2005 and joined into a plasmid pAMVCAT, which was similarly digested, to replace the nopaline synthase terminator therein. The plasmid pAMVCAT is similar to pAMVBTS except for the coding sequence being that for chloramphenicol transferase gene from Tn5 modified so as to remove an internal Nco I site.

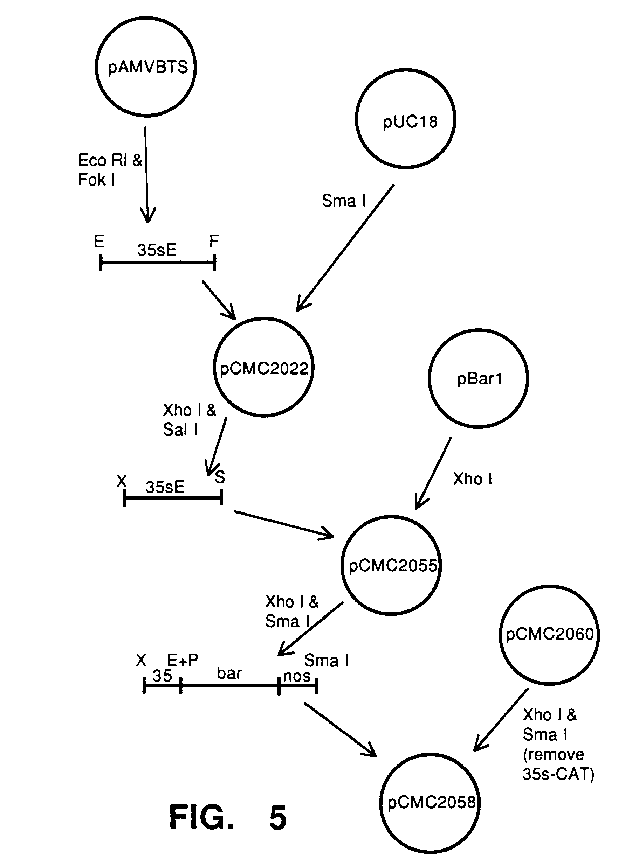

The Bar gene sequence itself was altered by adding an additional CaMV35S promoter-enhancer fragment to the 5' end of the chimeric Bar gene. This process is illustrated in Fig. 5. The CaMV35S enhancer fragment was obtained by isolating a 367 base pair fragment from Eco RI and Fok I digestion of pAMVBTS and filling in the blunt ends with Klenow polymerase. The blunt-ended fragment was then inserted into the Sma I site of pUC18 in an orientation where the Eco RI end of the inserted fragment is closest to the Kpn I site from pUC18 and the Fok I site is closest to the Bam HI site of pUC18. This plasmid, designated pCMC2022, was then digested with Xho I and Sal I and a 367 base pair fragment isolated and then inserted into pBar 1 which had been digested with Xho I. The resulting plasmid was designated pCMC2055. The plasmid pCMC2055 thus possessed, in order: an Xho I site; the CaMV35S enhancer; an Xba I site; a fusion of Xho I and Sal I sites; the CaMV35S promoter; the AMV 5' non-coding leader sequence; an Nco I site; the Bar coding region; an Sma I site; and the nopaline synthase 3' terminator. By digestion with Xho I and Sma I, and isolation of the 1400 base pair fragment, the chimeric gene was isolated without the polyadenylation terminator. This fragment was then ligated into pCMC2060 digested with Xho I and Sma I so that the promoter-CAT gene sequence was replaced with the enhancer-promoter-Bar gene sequence. This plasmid, designated pCMC2058, had, in sequence: the Xho I site; the CaMV35S enhancer; the Xba I site; the CaMV35S promoter; the AMV leader; the Nco I site; the Bar coding sequence; the Sma I site; the soybean ssu terminator; and Xba I and Sal I sites. The Xba I fragment from this plasmid thus represents an entire chimeric plant expression gene construction effective to express Bar in soybean cells.

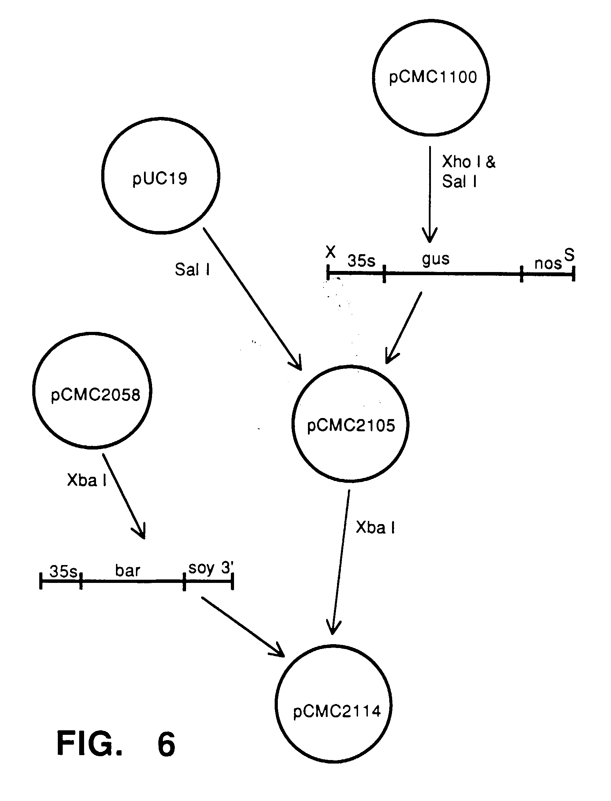

The above Bar gene construction was then added to another construction containing the gene for beta-glucuronidase (gus), as illustrated in Fig. 6. A plasmid pCMC1100 contains a gus expression cassette found to express in soybean plants and cells. The gus gene construct was isolated from that plasmid by digestion with Xho I and Sal I and isolation of the 2604 base pair fragment. The fragment was then inserted into pUC19 which had been digested with Sal I. This plasmid, designated pCMC2105, contained the gus insert positioned with the Xho I end of the insert closest to the Hind III site of the pUC19 polylinker and the Sal I end of the insert closest to the Eco RI site of that polylinker.

The Bar gene construction described above was then isolated as a 1353 base pair fragment from the digestion of pCMC2058 with Xba I. The fragment was inserted into the Xba I site of pCMC2105 to result in a plasmid designated pCMC2114. This plasmid includes the following sequences in this order: the Hind III site of the pUC19 polylinker; a Sal I and Xho I fusion; a CaMV35S promoter; an AMV 5' leader; the gus gene coding sequence; the nopaline synthase terminator; a Sal I site; an Xba I site; the soybean ssu terminator; the Sma site; the Bar coding sequence; the AMV 5' leader; the CaMV35S promoter; Xba I and Eco RI sites from the polylinker. The plasmid thus has two functional genes, gus and Bar, which transcribe towards each other, on a pUC19 plasmid. Both functional genes use the CaMV35S promoter and AMV 5' leader, but differ in their terminators.

### B. Transformation of Plants.

To prepare the DNA for transformation into plant tissues, the plasmid had to be coated onto the carrier particles. Aliquots of 1 micrograms of pCMC2114 and 1 microliter of 200 mM EDTA were added to 10 mg of 1 to 3 micron gold beads (Alfa Chemical Co.), mixed, and then dried under a stream of nitrogen. The particles with the DNA coated thereon were resuspended by adding 10 ml ethanol and grinding the particles to disperse them. The DNA-particle suspensions thus created were then coated onto 18 mm x 18 mm x 0.5 mil saran coated aluminized mylar carrier sheets by uniformly applying 163 microliters of suspension. After allowing the particles to settle on the carrier sheets for at least thirty seconds, the excess ethanol was drained away and the DNA and carrier particle coated sheets were allowed to air dry.

The actual transformation process employed was based on that disclosed in McCabe et al. Bio/Technology, 6:923-926 (1988) with some modifications including the use of mature dry seed, the use of aphidicolin to prepare the plant tissue, and the use of the Bar gene as a selectable marker to select for transformant tissues. The use of dry seed was for convenience. The aphidicolin was used to synchronize cell division by reversibly suspending DNA replication prior to foreign DNA insertion. The Bar gene allowed for effective herbicide selection of plant tissues.

In detail, seeds of soybean variety Williams were surface sterilized by soaking in 2% sodium hypochlorite for five minutes with stirring followed by five successive washings in distilled water. The seeds were then plated with their hilum end in contact with a medium containing 1% water agar, 10 mg/l aphidicolin in 0.1% DMSO, and CCB (0.4 g/l carbennicillin, 0.1 g/l cefotaxime, and 0.1 g/l benomyl) for one day. Then the seed coat and both cotyledons were removed to expose the embryonic axes. The ligules and primary leaves were then removed and the embryonic axes were plated radicle first into a basal MS media as modified as described in Barwhale et al., Planta, 167:473-481 (1986) with the further addition of 10 mg/l aphidicolin in 0.1% DMSO. This media, designated as OR + aphidicolin, contained a high level of benzylaminopurine (13.3 uM). Again the aphidicolin is intended to suspend DNA replication prior to to the transformation so that the cells would be synchronized in stage (Sala et al., Meth. Ent. 118, 87-96 (1986)). The axes were held on the OR + aphidicolin media overnight at 25 degrees, the first 6 hours in dark followed by 12 hours in light. The embryonic axes were then removed from the media and arranged on a target plate using 12% xantham glue and the CCB to hold the axes on a target surface and in a suitable position to allow particle acceleration of DNA into the meristematic tissues.

The DNA of plasmid pCMC2114, as prepared above, on the carrier sheet 22 was placed on the opening of the spark discharge chamber of the apparatus of Fig. 1. The spark discharge was performed at 16 KV in two separate treatments. In the first treatment, the DNA on particles were accelerated into the meristematic embryonic axes after removal from the 12 hour incubation in the light. The axes were then returned to the dark for 5 to 6 hours and then treated with pCMC2114 DNA injection again. After the second treatment, the axes were plated on a regeneration or shoot inducing MSR medium (a similar basal medium except benzylaminopurine is at 1.7 uM and no aphidicolin) and incubated in the dark for 2 days. The axes, again on MSR medium, were then transferred to a 16 hour light/8 hour dark cycle until shoots appeared, usually at 5 to 6 weeks. There were typically 3 to 6 shoots per embryonic axes explants.

When the individual shoots reached lengths of 1.5 to 2 cm, they were severed at their base. A 2 to 3 mm basal segment from each severed shoot was assayed for expression of the Gus gene. This assay was conducted by incubating the stem segment in 1 mM X-gluc (Clontech Labs), 0.1 M NaPO₄ (pH 7.0), and 0.5 mM potassium ferrocyamide at 37 degrees C overnight. The shoots were then cleared by boiling in lactophenol and examined for blue deposits indicating gus activity. One chimeric shoot was sufficiently transformed to express the gus gene well. The gus expressing shoot was grafted onto a soybean seedling to assist in growth of a soybean plant and this plant was later found to be expressing Bar as well.

The remainder of each of the shoots excised from the embryonic axes were placed on a 1/2 strength MS media with CCB and 3 mg/l bialaphos. The shoots were cultured on the selective medium for about 1 week. After about 7 to 10 days on 3 mg/l bialaphos, all nontransformed shoots had bleached and died. Several shoots appeared, however, to be transformed and were green and healthy throughout cultivation on the selection medium. The transformed shoots were then also grafted onto healthy soybean rootstock to assist in rapid growth.

To confirm the expression of the Bar gene in the selected shoots, about 5 to 10 mg of leaf or shoot tissue was removed from each plant and the total RNA from the tissue was isolated by the procedure described in Chomczynski and Sacchi, Analy. Biochem., 162:156-159 (1987). This isolated RNA was then analyzed for the presence of the Bar gene RNA using an RNA polymerase chain reaction (PCR) assay as described in Kawasaki et al., PNAS, 85:5698-5702 (1988), and as follows. The isolated RNA was resuspended in 10 µl water, and the suspended RNA was then used in a 50 µl reverse transcription assay to make cDNA. This reaction used the RNA, 50 mM KCl, 20mM Tris-HCL, pH7.5, 2.5 mM MgCl₂, 100 ug/ml bovine serum albumin, 1 mM each dATP, dGTP, dTTP and dCTP, 10-50 pmol DR46, and 100 units MuLV reverse transcriptase. DR46 was a single stranded oligonucleotide of the sequence 5'-CCCTGCAGTTACTATCAGAT-3'. After incubation at 42 degrees C for 30 minutes, the reaction was heated to 95 degrees C for 5 minutes. Then 25 ul of the cDNA reaction mix was used in a PCR reaction to amplify the Bar gene sequence in the cDNA. The PCR method is described in Saiki et al., Science, 230:1350-1354(1985). The PCR reaction was carried out using two oligonucleotide primers specific to Bar gene DNA, DR45 and DR46 (sequence above). The sequence of DR45 was 5'TACATCGAGACAAGCACGGTC-3'. The amplified region is a 484 base pair product. The results of this assay verified the presence and proper transcription of the Bar gene in at least sectors of the transformed shoots.

The expression of the Bar enzyme was confirmed by assaying for the enzyme itself using the procedure of DeBlock et al., EMBO 6:2513-2518 (1987). The assay confirmed the proper expression of the enzyme in the tissues of the shoots. It is presumed that the shoots were chimeric, possessing some cells or sectors transformed by the Bar gene and other which are non-transformed.

The following plasmids have been deposited with the American Type Culture Collection, 12301 Parklawn Drive, Rockville, MD, USA, and with the Cetus Master Culture Collection, Emeryville, CA, USA, under the following accession numbers:

| Plasmid | ATCC Accession No. | ATCC Deposit Date | CMCC Accession No. |
|---|---|---|---|
| pCMC1100 | 67641 | March 1, 1988 | 3290 |
| pAMVBTS | 53637 | June 24, 1987 | 3137 |
| pCMC2114 | 67936 | April 26, 1989 | 3575 |

The above deposits have been made under the terms of the Budapest Treaty.

The present invention is not to be limited in scope to the microorganisms deposited, since the deposited embodiment is but a single illustration of the present invention. Various modifications of the invention in addition to those disclosed herein will become apparent to one of ordinary skill in the art from the specification which still falls within the scope of the appended claims. It is also to be understood that all nucleotide sizes are approximate and are used for the purposes of description only.

## Claims

1. A method of genetically engineering a soybean plant for resistance to a glutamine synthetase inhibitor comprising the steps of
(a) isolating a protein coding sequence from a gene for resistance to a glutamine synthetase inhibitor from a microorganism;
(b) joining the protein coding sequence to a flanking regulatory sequence to make a chimeric gene construction effective to express the coding sequence in soybean cells;
(c) coating copies of the chimeric gene construction onto small carrier particles;
(d) physically accelerating the small carrier particles with the chimeric gene construction thereon into soybean tissue which can be cultivated into whole soybean plants;
(e) cultivating the soybean tissue on a medium containing an effective quantity of a glutamine synthetase inhibitor sufficient to be toxic to soybean tissue which is not transformed;
(f) cultivating the surviving tissue into whole soybean plants; and
(g) verifying the presence of the resistance trait in the soybean plants.

2. A method according to claim 1 wherein the protein coding sequence is for the enzyme phosphinothricin acetyltransferase.

3. A method according to claim 2 wherein the protein coding sequence is from the fungus Streptomyces hygroscopicus.

4. A method according to any one of the preceding claims wherein the flanking regulatory sequence includes a promoter, a non-translated expression enhancer located 5' to the coding sequence and a polyadenylation sequence located 3' to the coding sequence.

5. A method according to any one of the preceding claims wherein the small carrier particles are 1 to 3 µm gold beads.

6. A method according to any one of the preceding claims wherein the step of physically accelerating the particles is performed on an apparatus in which the propulsive force is provided by a spark discharge of electrical energy, with the voltage of the discharge being adjustable to adjust the force by which the particles are accelerated.

7. A method according to any one of the preceding claims wherein in step (e) the medium contains bialaphos, which has the structure:

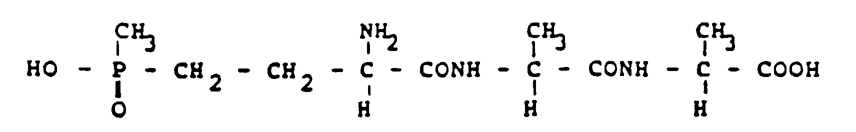

8. A method according to claim 7 wherein the bialaphos is present in a concentration of about 3mg/l of the medium.

9. A method according to any one of the preceding claims wherein the soybean tissue used in step (d) is meristematic tissue of soybean embryos.

10. A method according to claim 9 wherein the treated soybean embryos are cultured on a shoot-inducing medium to induce multiple shoot formation and wherein the shoots thus obtained are exposed to selection for resistance to the glutamine synthetase inhibitor.

11. A soybean plant comprising in its genome an inheritable trait for resistance to a glutamine synthetase inhibitor from a microorganism.

12. A soybean plant according to claim 11 comprising in its genome the Bar gene from Streptomyces hygroscopicus.

13. A soybean plant according to claim 11 or 12 that is not a plant of a plant variety.

14. Seeds of the soybean plant claimed in claim 11 or 13 and comprising in their genome a gene for resistance to a glutamine synthetase inhibitor from a microorganism or of the soybean plant claimed in claim 12 and comprising in their genome the Bar gene from Streptomyces hygroscopicus.

## Patentansprüche

1. Verfahren zur Erzeugung einer Resistenz gegen einen Glutamin-Synthetase-Inhibitor durch genetische Veränderung einer Sojabohnen-Pflanze umfassend Schritte, bei denen man
(a) eine Protein kodierende Sequenz eines Glutamin-Synthethase-Inhibitor Resistenzsgens von einem Mikroorganismus isoliert;
(b) die Protein kodierende Sequenz mit flankierende regulatorische Sequenzen verknüpft, um ein chimeres Genkonstrukt zu erzeugen, welches die Expression der kodierenden Sequenz in Sojabohnen-Zellen bewirkt;
(c) Kopien des chimeren Genkonstruktes auf kleine Trägerpartikel schichtet;
(d) die kleinen Trägerpartikel mit dem chimeren Genkonstrukt darauf physisch in Gewebe von Sojabohnen beschleunigt, welches zu ganzen Sojabohnen-Pflanzen gezogen werden kann;
(e) Sojabohnen-Gewebe auf einem Medium zieht, welches eine ausreichende Menge eines Glutamin-Synthetase-Inhibitors aufweist, um toxisch auf nicht transformiertes Sojabohnen-Gewebe zu wirken;
(f) überlebendes Gewebe zu ganzen Sojabohnen-Pflanzen zieht; und
(g) die Gegenwart des Resistenzmerkmals in den Sojabohnen-Pflanzen verifiziert.

2. Verfahren gemäß Anspruch 1, bei dem die Protein kodierende Sequenz für das Enzym Phosphinotricin-Acetyltransferase kodiert.

3. Verfahren nach Anspruch 2, bei dem die Protein kodierende Sequenz von dem Pilz Streptomyces hygroscopius abstammt.

4. Verfahren nach einem der vorstehenden Ansprüche, bei dem die flankierende regulatorische Sequenz im 5'-Bereich der kodierenden Sequenz einen Promotor, einen nicht-translatierten Expressions-Enhancer und im 3'-Bereich der kodierenden Sequenz eine Polyadenylierungssequenz umfaßt.

5. Verfahren nach irgendeinem der vorstehenden Ansprüche, bei dem die kleinen Trägerpartikel Goldkügelchen mit 1 bis 3 µm sind.

6. Verfahren nach irgendeinem der vorstehenden Ansprüche, bei dem man den Schritt der physischen Beschleunigung der Partikel mittels eines Gerätes durchführt, in dem die beschleunigende Kraft durch Funkenentladung einer elektrischen Energiequelle erzeugt wird, wobei die Spannung der Entladung wählbar ist, um die Kraft der Partikelbeschleunigung einzustellen.

7. Verfahren nach irgendeinem der vorstehenden Ansprüche, bei dem das Medium in Schritt (e) Bialaphos enthält, welches folgende Struktur aufweist:

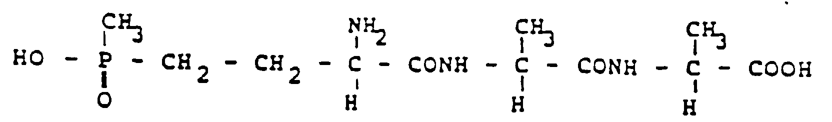

8. Verfahren nach Anspruch 7, bei dem Bialaphos im Medium in einer Konzentration von etwa 3 mg/l vorliegt.

9. Verfahren nach irgendeinem der vorstehenden Ansprüche, bei dem man in Schritt (d) meristematisches Gewebe von Sojabohnen-Embryonen als Sojabohnen-Gewebe verwendet.

10. Verfahren nach Anspruch 9, bei dem man die behandelten Sojabohnen-Embryonen auf einem Trieb-induzierenden Medium zieht, um multiple Triebbildung zu induzieren, und die so erhaltenen Triebe einer Selektion auf Resistenz gegen den Glutamin-Synthetase-Inhibitor aussetzt.

11. Sojabohnen-Pflanze, dessen Genom ein vererbbares Merkmal von einem Mikroorganismus umfaßt, das Resistenz gegen einen Glutamin-Synthetase-Inhibitor verleiht.

12. Sojabohnen-Pflanze nach Anspruch 11, dessen Genom das Bar-Gen von Streptomyces hygroscopius umfaßt.

13. Sojabohnen-Pflanze nach Anspruch 11 oder 12, die keine Pflanze einer Pflanzensorte ist.

14. Samen der Sojabohnen-Pflanze gemäß Anspruch 11 oder 13, deren Genom ein Gen von einem Mikroorganismus umfaßt, das Resistenz gegen einen Glutamin-Synthetase-Inhibitor verleiht, oder von einer Sojabohnen-Pflanze gemäß Anspruch 12, deren Genom das Bar-Gen von Streptomyces hygroscopius umfassen.

## Revendications

1. Méthode de manipulation génétique d'un plant de soja pour la résistance à l'inhibiteur de glutamine synthétase, comprenant les étapes de :
(a) isoler une séquence codant pour une protéine d'un gène pour la résistance à un inhibiteur de glutamine synthétase d'un microorganisme;
(b) joindre la séquence de codage pour la protéine à une séquence régulatrice adjacente pour produire une construction de gène chimérique efficace pour exprimer la séquence de codage dans des cellules de soja.
(c) enduire des copies de la construction du gène chimérique sur de petites particules porteuses;
(d) accélérer physiquement les petites particules porteuses avec la construction du gène chimérique par dessus dans du tissu de soja qui peut être cultivé en plants de soja entiers;
(e) cultiver le tissu de soja sur un milieu contenant une quantité efficace d'un inhibiteur de glutamine synthétase, suffisante pour être toxique pour le tissu de soja qui n'est pas transformé;
(f) cultiver le tissu survivant en plants de soja entiers; et
(g) vérifier la présence du trait de résistance dans les plants de soja.

2. Méthode selon la revendication 1 où la séquence de codage de la protéine est pour l'enzyme phosphinothricine acétyltransférase.

3. Méthode selon la revendication 2 où la séquence de codage de la protéine est du champignon Streptomyces hygroscopicus.

4. Méthode selon l'une quelconque des revendications précédentes où la séquence régulatrice adjacente comprend un promoteur, un enrichisseur d'expression non traduit placé côté 5' par rapport à la séquence de codage et une séquence de polyadénylation placée côté 5' par rapport à la séquence de codage.

5. Méthode selon l'une quelconque des revendications précédentes où les petites particules porteuses sont des perles d'or de 1 à 3 µm.

6. Méthode selon l'une quelconque des revendications précédentes où l'étape d'accélérer physiquement les particules est accomplie sur un appareil dans lequel la force de propulsion est produite par une décharge d'une étincelle d'énergie électrique, avec la tension de la décharge qui est réglable pour ajuster la force par laquelle les particules sont accélérées.

7. Méthode selon l'une quelconque des revendications précédentes où, à l'étape (e), le milieu contient du bialaphos, qui a la structure:

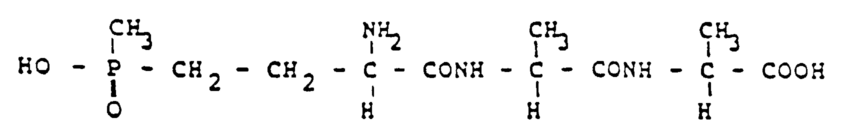

8. Méthode selon la revendication 7 où le bialaphos est présent à une concentration d'environ 3 mg/ml du milieu.

9. Méthode selon l'une quelconque des revendications précédentes où le tissu de soja utilisé à l'étape (d) est le tissu du méristème d'embryons de soja.

10. Méthode selon la revendication 9 où les embryons traités de soja sont cultivés sur un milieu induisant les pousses pour induire la formation de pousses multiples et où les pousses ainsi obtenues sont exposées à une sélection pour la résistance à l'inhibiteur de la glutamine synthase.

11. Plant de soja comprenant, dans son génome, un trait héritable pour la résistance à l'inhibiteur de glutamine synthétase d'un microorganisme.

12. Plant de soja selon la revendication 11 comprenant, dans son génome, le gène Bar de Streptomyces hygroscopicus.

13. Plant de soja selon la revendication 11 ou 12 qui n'est pas un plant d'une variété de plants.

14. Graines du plant de soja selon la revendication 11 ou 13 et comprenant, dans leur génome, un gène pour la résistance à un inhibiteur de glutamine synthétase provenant d'un microorganisme ou du plant de soja revendiqué à la revendication 12 et comprenant dans leurs génomes le gène Bar de Streptomyces hygroscopicus.
